(19) Europäisches Patentamt
European Patent Office
Office européen des brevets

(11) Numéro de publication : **0 136 198**
**B1**

(12) **FASCICULE DE BREVET EUROPÉEN**

(45) Date de publication du fascicule du brevet : **10.02.88**

(51) Int. Cl.⁴ : **C 07 D487/04**, A 61 K 31/505 // (C07D487/04, 249:00, 239:00)

(21) Numéro de dépôt : **84401551.1**

(22) Date de dépôt : **24.07.84**

(54) **Dérivés de triazolo pyrimidine, leur procédé de préparation et leur application thérapeutique en tant que toni-cardiaques.**

(30) Priorité : **25.07.83 FR 8312443**

(43) Date de publication de la demande : **03.04.85 Bulletin 85/14**

(45) Mention de la délivrance du brevet : **10.02.88 Bulletin 88/06**

(84) Etats contractants désignés : **AT BE CH DE FR GB IT LI LU NL SE**

(56) Documents cités :
**GB-A- 845 552**
**CHEMICAL ABSTRACTS, vol. 49, no. 21, 10 novembre 1955, colonne 14544h, Columbus, Ohio, USA; K. MUROBUSHI et al.: "Effect of azaindolizines on the photographic emulsions" & J. CHEM. SOC. JAPAN, IND. CHEM. SECT. 58, 440-441, 1955**
**"Dictionnaire encyclopédique LAROUSSE 1979 page 45".**
**"Dictionnaire de la chimie et de ses applications 3. édition page 46, 1978**

(73) Titulaire : **SANOFI**
**40, Avenue George V**
**F-75008 Paris (FR)**

(72) Inventeur : **Barthelemy, Gérard**
**1400 Chemin de Couloume Seysses**
**F-31600 Muret (FR)**
Inventeur : **Vallat, Jean-Noel**
**72 Rue Alfred Duméril**
**F-31400 Toulouse (FR)**
Inventeur : **Hallot, André**
**83 Rue du Juge**
**F-34980 Saint-Gely-du-Sesc (FR)**

(74) Mandataire : **Polus, Camille et al**
**c/o Cabinet Lavoix 2, Place d'Estienne d'Orves**
**F-75441 Paris Cedex 09 (FR)**

**EP 0 136 198 B1**

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

Jouve, 18, rue St-Denis, 75001 Paris, France

## Description

GB-A-845 552 décrit des dérivés de triazolo-pyrimidine utilisables notamment comme antitumoraux.

La présente invention est relative à de nouvelles triazolo-pyrimidines, à leur procédé de préparation et à leur application en médecine humaine et vétérinaire.

Les nouveaux composés selon l'invention répondent à la formule suivante :

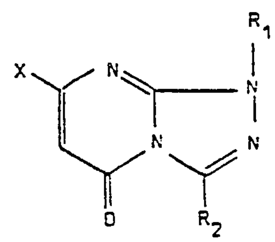

(I)

dans laquelle X représente un noyau pyridyl -2, -3 ou -4 éventuellement substitué par au moins un groupe alcoyle ou alcoxy en $C_1$ à $C_4$ à chaîne droite ou ramifiée, hydroxy ou cyano, $R_1$ représente l'hydrogène ou un groupe alcoyle en $C_1$ à $C_5$, à chaîne droite ou ramifiée, allyle ou propargyle et $R_2$ représente un groupe alcoyle en $C_1$ à $C_4$ à chaîne droite ou ramifiée, ainsi que les sels d'addition avec les acides minéraux ou organiques pharmaceutiquement acceptables.

L'invention a également pour objet un procédé de préparation des composés précités, caractérisé en ce qu'on condense, dans un solvant organique, un dérivé de formule (II) :

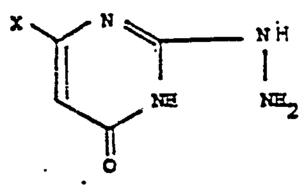

(II)

dans lequel X est tel que défini ci-dessus, avec des ortho esters de formule générale

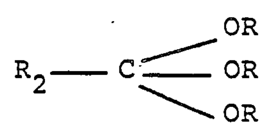

(III)

dans lesquels $R_2$ est tel que défini ci-dessus et R représente le groupe méthyle ou éthyle et on procède ensuite éventuellement à une alcoylation, en présence d'une base dans un solvant organique, par les méthodes classiques si l'on veut obtenir les composés dans lesquels $R_1$ n'est pas l'hydrogène.

La réaction de condensation entre les composés des formules II et III s'effectue dans un solvant organique. Ce solvant peut être l'ortho ester lui-même (III) et dans ce cas, il faut opérer à une température de 95 °C. Ce solvant peut être encore le xylène, le butanol ou le toluène et la réaction s'effectue à reflux.

La réaction d'alcoylation s'effectue par l'action d'un halogénure d'alcoyle de formule Hal—$R_1$, Hal représentant le chlore, le brome ou l'iode et $R_1$ est tel que défini ci-dessus, en présence d'une base qui peut être l'hydrure de sodium ou l'amidure de sodium dans des solvants tels que le diméthyl formamide, le diméthyl sulfoxyde, l'hexaméthyl phosphorotriamide, l'acétonitrile ou le toluène, à des températures comprises entre 25 °C et 70 °C.

Dans les cas où $R_1$ représente un radical méthyle ou éthyle, on peut utiliser comme agent d'alcoylation le diméthyl sulfate ou le diéthyl sulfate en présence de potasse à des températures comprises entre 25 °C et 50 °C (Allen et al., J. Org. Chem. 25, 361, 1960).

L'alcoylation de ces composés s'effectue aussi par l'action d'un halogénure d'alcoyle en présence de fluorure de tétrabutylammonium à température ambiante.

Les sels des composés de formule I sont ensuite préparés selon les méthodes classiques.

Les composés de formule II sont préparés selon la méthode décrite par D. Libermann et A. Rouaix (C. R., 240, 984-986, 1955) et Y. Minoru et al., (Yakugaku Zasshi 96/9, 1094-102, 1976).

Les exemples suivants illustrent l'invention :

## Exemple 1

méthyl-3 oxo-5 (pyridyl-3)-7 triazolo [4,3-a] pyrimidine (1H) (X = pyridyl-3, $R_1$ = H, $R_2$ = $CH_3$) (dérivé N° 1)

On mélange dans un erlenmeyer 23 g (0,113 mole) de hydrazino-2 oxo-4 (pyridyl-3)-6 pyrimidine (composé de formule II) et 73,6 g d'orthoacétate d'éthyle et 500 ml de n-butanol. On chauffe pendant 4 heures à reflux, puis on filtre et on lave le précipité à l'éther. On recueille 24 g de cristaux (rendement 93 %). C. C. M. Acétate d'éthyle-méthanol (90-10).

Point de fusion > 260 °C.

## Exemple 2

méthyl-3 oxo-5 (pyridyl-2)-7 triazolo [4,3-a] pyrimidine (1H) (X = pyridyl-2, $R_1$ = H, $R_2$ = $CH_3$) (dérivé N° 2)

Selon le mode opératoire décrit à l'exemple 1, on prépare ce composé à partir de l'hydrazino-2 oxo-4 (pyridyl-2)-6 pyrimidine et de l'orthoacétate d'éthyle par chauffage pendant 4 h à 95 °C. On obtient des cristaux blancs (rendement 91 %).

Point de fusion > 260 °C.

## Exemple 3

méthyl-3 oxo-5 (pyridyl-4)-7 triazolo [4,3-a] pyrimidine (1H) (X = pyridyl-4, $R_1$ = H, $R_2$ = $CH_3$) (dérivé N° 3).

Selon le mode opératoire décrit à l'exemple 1, on prépare ce composé à partir de l'hydrazino-2 oxo-4 (pyridyl-4)-6 pyrimidine et de l'orthoacétate d'éthyle par chauffage à reflux pendant 3 heures. On recueille des cristaux jaune clair (rendement 83 %).

Point de fusion > 260 °C.

## Exemple 4

éthyl-3 oxo-5 (pyridyl-2)-7 triazolo [4,3-a] pyrimidine (1H) (X = pyridyl-2, $R_1$ = H, $R_2$ = $CH_2—CH_3$) (dérivé N° 4).

Selon le mode opératoire décrit à l'exemple 1, on prépare ce composé à partir de l'hydrazino-2 oxo-4 (pyridyl-2)-6 pyrimidine dont le point de fusion est de 250 °C et de l'ortho propionate d'éthyle par chauffage à reflux pendant 2 heures. On obtient des cristaux blancs (rendement 85 %).

Point de fusion > 260 °C.

## Exemple 5

éthyl-3 oxo-5 (pyridyl-3)-7 triazolo [4,3-a] pyrimidine (1H) (X = pyridyl-3, $R_1$ = H, $R_2$ = $CH_2—CH_3$) (dérivé N° 5).

Selon le mode opératoire décrit à l'exemple 1, on prépare ce composé à partir de l'hydrazino-2 oxo-4 (pyridyl-3)-6 pyrimidine et de l'ortho propionate d'éthyle par chauffage à reflux pendant 2 heures. On obtient des cristaux jaune clair (rendement 63 %).

Point de fusion > 260 °C.

## Exemple 6

éthyl-3 oxo-5 (pyridyl-4)-7 triazolo [4,3-a] pyrimidine (1H) (X = pyridyl-4, $R_1$ = H, $R_2$ = $CH_2—CH_3$) (dérivé N° 6).

Selon le mode opératoire décrit à l'exemple 1, on prépare ce composé à partir de l'hydrazino-2 oxo-4 (pyridyl-4)-6 pyrimidine et de l'ortho propionate d'éthyle par chauffage à reflux pendant 2 heures. On obtient des cristaux beiges (rendement 42 %).

Point de fusion > 260 °C.

## Exemple 7

éthyl-1 méthyl-3 oxo-5 (pyridyl-3)-7 triazolo [4,3-a] pyrimidine (X = pyridyl-3, $R_1$ = $CH_2—CH_3$, $R_2$ = $CH_3$) (dérivé N° 7).

Dans un ballon contenant 1,36 g (0,028 mole) d'hydrure de sodium à 50 % en suspension dans 50 ml de diméthyl formamide (DMF) sous atmosphère d'azote, on introduit goutte à goutte une solution de 6,4 g (0,028 mole) de méthyl-3 oxo-5 (pyridyl-3)-7 triazolo [4,3-a] pyrimidine (1H) (dérivé N° 1) dans 400 ml de DMF. A la fin du dégagement d'hydrogène, on chauffe à 70 °C et on ajoute lentement 2,2 ml (0,028 mole) de bromure d'éthyle, en maintenant le mélange pendant 2 heures à cette température. On élimine le DMF, on reprend à l'eau puis on extrait par de l'acétate d'éthyle. On sèche ensuite la solution organique sur le sulfate de magnésium et on concentre et on recristallise dans l'heptane. On obtient des cristaux blancs cotonneux (rendement 69 %).

C. C. M. acétate d'éthyle.

Point de fusion : 144 °C.

Pour obtenir le méthane sulfonate, on mélange 5 g (0,019 6 mole) du composé obtenu, 20 ml d'eau et 1,88 g (0,019 6 mole) d'acide méthane sulfonique. Par lyophilisation de la solution, on obtient une poudre qui, après lavage à l'éther est séchée sous vide. On obtient 6,9 g d'une poudre blanche (rendement 98,5 %). Le sel obtenu recristallise avec une demie molécule d'eau et son point de fusion est de 208 °C.

## Exemple 8

éthyl-1 méthyl-3 oxo-5 (pyridyl-2)-7 triazolo [4,3-a] pyrimidine (X = pyridyl-2, $R_1 = CH_2—CH_3$, $R_2 = CH_3$) (dérivé N° 8).

Selon le mode opératoire décrit à l'exemple 7, on prépare ce composé à partir du méthyl-3 oxo-5 (pyridyl-2)-7 triazolo [4,3-a] pyrimidine (1H) et du bromure d'éthyle. On recueille des cristaux cotonneux orange (rendement 72 %).

Point de fusion : 156 °C.

## Exemple 9

éthyl-1 méthyl-3 oxo-5 (pyridyl-4)-7 triazolo [4,3-a] pyrimidine (X = pyridyl-4, $R_1 = CH_2—CH_3$, $R_2 = CH_3$) (dérivé N° 9).

Selon le mode opératoire décrit à l'exemple 7, on prépare ce composé à partir du méthyl-3 oxo-5 (pyridyl-4)-7 triazolo [4,3-a] pyrimidine (1H) et du bromure d'éthyle. On obtient des cristaux blanchâtres (rendement 53 %).

Point de fusion : 160 °C-162 °C.

## Exemple 10

méthyl-1 méthyl-3 oxo-5 (pyridyl-2)-7 triazolo [4,3-a] pyrimidine (X = pyridyl-2, $R_1 = CH_3$, $R_2 = CH_3$) (dérivé N° 10).

Selon le mode opératoire décrit à l'exemple 7, on prépare ce composé à partir du méthyl-3 oxo-5 (pyridyl-2)-7 triazolo [4,3-a] pyrimidine (1H) et de l'iodure de méthyle. On obtient des cristaux blancs (rendement 81 %).

Point de fusion : 211 °C.

## Exemple 11

méthyl-1 méthyl-3 oxo-5 (pyridyl-4)-7 triazolo [4,3-a] pyrimidine (X = pyridyl-4, $R_1 = CH_3$, $R_2 = CH_3$) (dérivé N° 11).

Selon le mode opératoire décrit à l'exemple 7, on prépare ce composé à partir du méthyl-3 oxo-5 (pyridyl-4)-7 triazolo [4,3-a] pyrimidine (1H) et de l'iodure de méthyle. On obtient des cristaux beiges (rendement 50 %).

Point de fusion : 200 °C-202 °C.

## Exemple 12

propyl-1 méthyl-3 oxo-5 (pyridyl-2)-7 triazolo [4,3-a] pyrimidine (X = pyridyl-2, $R_1 = CH_2—CH_2—CH_3$, $R_2 = CH_3$) (Dérivé N° 12).

Selon le mode opératoire décrit à l'exemple 7, on prépare ce composé à partir du méthyl-3 oxo-5 (pyridyl-2)-7 triazolo [4,3-a] pyrimidine (1H) et de l'iodure de propyle. On obtient des cristaux blancs (rendement 80 %).

Point de fusion : 124 °C.

### Exemple 13

méthyl-1 éthyl-3 oxo-5 (pyridyl-2)-7 triazolo [4,3-a] pyrimidine (X = pyridyl-2, $R_1 = CH_3$, $R_2 = CH_2—CH_3$) (Dérivé N° 13)

Selon le mode opératoire décrit à l'exemple 7, on prépare ce composé à partir de l'éthyl-3 oxo-5 (pyridyl-2)-7 triazolo [4,3-a] pyrimidine (1H) et de l'iodure de méthyle. On obtient des cristaux blancs (rendement 70 %).

Point de fusion : 172 °C.

### Exemple 14

propyl-1 éthyl-3 oxo-5 (pyridyl-2)-7 triazolo [4,3-a] pyrimidine (X = pyridyl-2, $R_1 = CH_2—CH_2—CH_3$, $R_2 = CH_2—CH_3$) (Dérivé N° 14).

Selon le mode opératoire décrit à l'exemple 7, on prépare ce composé à partir de l'éthyl-3 oxo-5 (pyridyl-2)-7 triazolo [4,3-a] pyrimidine (1H) et de l'iodure de propyle. On obtient des cristaux blancs (rendement 85 %).

Point de fusion : 153 °C.

### Exemple 15

éthyl-1 méthyl-3 oxo-5 (butyl-5 pyridyl-2)-7 triazolo [4,3-a] pyrimidine (X = butyl-5 pyridyl-2, $R_1 = CH_2—CH_3$, $R_2 = CH_3$) (Dérivé N° 15).

Selon le mode opératoire décrit à l'exemple 7, on prépare ce composé à partir de méthyl-3 oxo-5 (butyl-5 pyridyl-2)-7 triazolo [4,3-a] pyrimidine (1H) et de bromure d'éthyle. On obtient des cristaux blancs (rendement 82 %).

Point de fusion : 106 °C.

### Exemple 16

méthyl-1 méthyl-3 oxo-5 (pyridyl-3)-7 triazolo [4,3-a] pyrimidine méthane sulfonate (X = pyridyl-3, $R_1 = CH_3$, $R_2 = CH_3$) (Dérivé N° 16).

Selon le mode opératoire décrit à l'exemple 7, on prépare ce composé à partir de méthyl-3 oxo-5 (pyridyl-3)-7 triazolo [4,3-a] pyrimidine (1H) et de l'iodure de méthyle. On prépare ensuite le sel à l'aide de l'acide méthane sulfonique. On obtient des cristaux blanchâtres (rendement 62 %).

Point de fusion de la base : 208 °C.

### Exemple 17

propyl-1 méthyl-3 oxo-5 (pyridyl-3)-7 triazolo [4,3-a] pyrimidine méthane sulfonate (X = pyridyl-3, $R_1 = CH_2—CH_2—CH_3$, $R_2 = CH_3$) (Dérivé N° 17).

Selon le mode opératoire décrit à l'exemple 7, on prépare ce composé à partir du méthyl-3 oxo-5 (pyridyl-3)-7 triazolo [4,3-a] pyrimidine (1H) et du bromure de propyle. On prépare ensuite le sel à l'aide de l'acide méthane sulfonique. On obtient des cristaux blancs (rendement 73 %).

Point de fusion : 108 °C.

### Exemple 18

pentyl-1 méthyl-3 oxo-5 (pyridyl-3)-7 triazolo [4,3-a] pyrimidine (X = pyridyl-3, $R_1 = (CH_2)_4—CH_3$, $R_2 = CH_3$) (Dérivé N° 18).

Selon le mode opératoire décrit à l'exemple 7, on prépare ce composé à partir du méthyl-3 oxo-5 (pyridyl-3)-7 triazolo [4,3-a] pyrimidine (1H) et du bromure de pentyle. On obtient des cristaux blancs (rendement 83 %).

Point de fusion : 97 °C.

### Exemple 19

éthyl-1 butyl-3 oxo-5 (pyridyl-3)-7 triazolo [4,3-a] pyrimidine (X = pyridyl-3, $R_1 = CH_2—CH_3$, $R_2 = (CH_2)_3—CH_3$) (Dérivé N° 19).

Selon le mode opératoire décrit à l'exemple 7, on prépare ce composé à partir du butyl-3 oxo-5 (pyridyl-3)-7 triazolo [4,3-a] pyrimidine (1H) et du bromure d'éthyle. On obtient des cristaux crème (rendement 35 %).

Point de fusion : 119 °C.

Exemple 20

butyl-3 oxo-5 (pyridyl-3)-7 triazolo [4,3-a] pyrimidine (1H) (X = pyridyl-3, $R_1$ = H, $R_2 = (CH_2)_3{-}CH_3$) (Dérivé N° 20).

Selon le mode opératoire décrit à l'exemple 1, on prépare ce composé à partir de l'hydrazino-2 oxo-4 (pyridyl-3) pyrimidine et de l'orthovalérate de méthyle. On obtient des cristaux blancs (rendement 76 %).

Point de fusion : > 250 °C.

Exemple 21

méthyl-1 butyl-3 oxo-5 (pyridyl-3)-7 triazolo [4,3-a] pyrimidine (X = pyridyl-3, $R_1 = CH_3$, $R_2 = (CH_2)_3{-}CH_3$) (Dérivé N° 21).

Selon le mode opératoire décrit à l'exemple 7, on prépare ce composé à partir du butyl-3 oxo-5 (pyridyl-3)-7 triazolo [4,3-a] pyrimidine (1H) et de l'iodure de méthyle. On obtient des cristaux blancs (rendement 74 %).

Point de fusion : 124 °C.

Exemple 22

propyl-1 méthyl-3 oxo-5 (pyridyl-4)-7 triazolo [4,3-a] pyrimidine (X = pyridyl-4, $R_1 = CH_2{-}CH_2{-}CH_3$, $R_2 = CH_3$) (Dérivé N° 22).

Selon le mode opératoire décrit à l'exemple 7, on prépare ce composé à partir du méthyl-3 oxo-5 (pyridyl-4)-7 triazolo [4,3-a] pyrimidine (1H) et du bromure de propyle. On obtient des cristaux blancs (rendement 58 %).

Point de fusion : 144 °C.

Exemple 23

(propyl-2)-1 méthyl-3 oxo-5 (pyridyl-4)-7 triazolo [4,3-a] pyrimidine (X = pyridyl-4, $R_1 = (CH_3)_2{-}CH$, $R_2 = CH_3$) (Dérivé N° 23).

Selon le mode opératoire décrit à l'exemple 7, on prépare ce composé à partir du méthyl-3 oxo-5 (pyridyl-4)-7 triazolo [4,3-a] pyrimidine (1H) et du chlorure d'isopropyle. On obtient des cristaux blancs (rendement 32 %).

Point de fusion : 170 °C.

Exemple 24

butyl-1 méthyl-3 oxo-5 (pyridyl-4)-7 triazolo [4,3-a] pyrimidine (X = pyridyl-4, $R_1 = (CH_2)_3{-}CH_3$, $R_2 = CH_3$) (Dérivé N° 24).

Selon le mode opératoire décrit à l'exemple 7, on prépare ce composé à partir du méthyl-3 oxo-5 (pyridyl-4)-7 triazolo [4,3-a] pyrimidine (1H) et du bromure de butyle. On obtient des cristaux blancs (rendement 85 %).

Point de fusion : 106 °C.

Exemple 25

(propyl-2-yl)-1 méthyl-3 oxo-5 (pyridyl-4)-7 triazolo [4,3-a] pyrimidine (X = pyridyl-4, $R_1 = CH_2{-}C = CH$, $R_2 = CH_3$) (Dérivé N° 25).

Selon le mode opératoire décrit à l'exemple 7, on prépare ce composé à partir du méthyl-3 oxo-5 (pyridyl-4)-7 triazolo [4,3-a] pyrimidine (1H) et du bromure de propargyle. On obtient des cristaux blancs (rendement 28 %).

Point de fusion : 178 °C.

Exemple 26

allyl-1 méthyl-3 oxo-5 (pyridyl-4)-7 triazolo [4,3-a] pyrimidine (X = pyridyl-4, $R_1 = CH_2—CH = CH_2$, $R_2 = CH_3$) (Dérivé N° 26).

Selon le mode opératoire décrit à l'exemple 7, on prépare ce composé à partir du méthyl-3 oxo-5 (pyridyl-4)-7 triazolo [4,3-a] pyrimidine (1H) et du bromure d'allyle. On obtient des cristaux blanchâtres (rendement 47 %).

Point de fusion : 146 °C.

Exemple 27

méthyl-1 éthyl-3 oxo-5 (pyridyl-4)-7 triazolo [4,3-a] pyrimidine méthane sulfonate (X = pyridyl-4, $R_1 = CH_3$, $R_2 = CH_2—CH_3$) (Dérivé N° 27).

Selon le mode opératoire décrit à l'exemple 7, on prépare ce composé à partir de l'éthyl-3 oxo-5 (pyridyl-4)-7 triazolo [4,3-a] pyrimidine (1H) et de l'iodure de méthyle. Le sel est ensuite préparé à l'aide de l'acide méthane sulfonique. On obtient des cristaux blancs (rendement 65 %).

Point de fusion : 190 °C.

Exemple 28

diéthyl-1,3 oxo-5 (pyridyl-4)-7 triazolo [4,3-a] pyrimidine (X = pyridyl-4, $R_1 = CH_2—CH_3$, $R_2 = CH_2—CH_3$) (Dérivé N° 28).

Selon le mode opératoire décrit à l'exemple 7, on prépare ce composé à partir de l'éthyl-3 oxo-5 (pyridyl-4)-7 triazolo [4,3-a] pyrimidine (1H) et du bromure d'éthyle. On obtient des cristaux blancs (rendement 81 %).

Point de fusion : 162 °C.

Exemple 29

propyl-1 éthyl-3 oxo-5 (pyridyl-4)-7 triazolo [4,3-a] pyrimidine, méthane sulfonate (X = pyridyl-4, $R_1 = CH_2—CH_2—CH_3$, $R_2 = CH_2—CH_3$) (Dérivé N° 29).

Selon le mode opératoire décrit à l'exemple 7, on prépare ce composé à partir de l'éthyl-3 oxo-5 (pyridyl-4)-7 triazolo [4,3-a] pyrimidine (1H) et du bromure de propyle. On obtient des cristaux beiges (rendement 59 %).

Point de fusion : 110 °C.

Les résultats des essais toxicologiques et pharmacologiques qui sont rapportés ci-après, ont mis en évidence les intéressantes propriétés des dérivés de l'invention, notamment une faible toxicité, une excellente tolérance ainsi qu'une activité toni-cardiaque.

L'invention a donc encore pour objet un médicament ayant en particulier une activité toni-cardiaque, caractérisé en ce qu'il contient, à titre de principe actif un dérivé de formule I.

Etude toxicologique

Les composés de l'invention bénéficient d'une excellente tolérance et d'une faible toxicité.

Au cours des essais de toxicité aiguë, chronique, subchronique ou retardée, effectués sur différentes espèces animales (souris, rat et lapin), les dérivés de l'invention ont été bien tolérés : il n'a pas été possible de mettre en évidence une quelconque anomalie ou perturbation dans les examens biochimiques, microscopiques effectués en cours ou en fin d'expérimentation.

Etude pharmacologique

L'activité toni-cardiaque du médicament de l'invention a été mise en évidence par l'étude de l'effet inotrope positif par les composés de l'invention.

Cette étude a été effectuée selon la technique de J. V. Levy (Methods in Pharmacology, Vol. 1, Schwartz).

Sur des cobayes albinos (Dunkin-Hartley) mâles et femelles, pesant de 600 g à 800 g, après rupture des vertèbres cervicales, ouverture du thorax et déchirement du péricarde, l'oreillette droite est rapidement prélevée et placée dans un bain nourricier.

Le produit à tester est ajouté dans la cuve à organe d'une contenance de 50 ml, sous un volume de 0,5 ml, après solubilisation dans l'acide chlorhydrique normal. Le pH final de la solution nourricière est relevé à la fin de chaque expérimentation.

Les paramètres suivants sont enregistrés sur un polygraphe :

la tension développée (myographe isométrique),

la vitesse de contraction et la vitesse de relaxation calculées par la dérivée de la tension par rapport au temps.

De la courbe de la tension développée, on détermine en outre la fréquence des contractions.

Les effets des dérivés à tester ont été recherchés à des doses allant de $10^{-7}$ à $10^{-3}$ mole/litre (M/L).

Les résultats qui sont rassemblés dans les tableaux suivants concernent les composés les plus actifs.

On constate que :

la tension développée est progressivement augmentée de $10^{-6}$ M/L avec un maximum d'effet à $2 \cdot 10^{-3}$ M/L pour le dérivé N° 7 (+ 197 %), $10^{-3}$ M/L pour le dérivé N° 9 (+ 129 %), le dérivé N° 12 (145 %), le dérivé N° 16 (130 %), le dérivé N° 17 (110 %) et le dérivé N° 27 (114 %),

la fréquence des contractions n'est pas modifiée par les différentes doses testées,

les vitesses de contraction et de relaxation suivent l'évolution de la tension développée.

La durée totale de l'ensemble des effets étant de 30 minutes.

Les études toxicologiques et pharmacologiques qui viennent d'être rapportées ont mis en évidence la faible toxicité des composés de l'invention et leur bonne tolérance, ainsi que leur intéressante activité toni-cardiaque qui les rendent très utiles en thérapeutique humaine et vétérinaire.

Chaque dose unitaire contient avantageusement de 0,050 g à 1,00 g de principe actif, les doses administrables journellement pouvant varier de 0,050 g à 3,00 g de principe actif en fonction de l'âge du patient et de la sévérité de l'affection traitée.

On donnera, ci-après, à titre d'exemples non limitatifs, quelques formulations pharmaceutiques du médicament de l'invention.

1) Comprimés dragéifiés

Dérivé N° 5 0,200 g

Excipient : amidon, sucre blanc officinal, carbonate de calcium, talc, stéarate de magnésium, gomme arabique, cire de carnauba.

2) Comprimés

Dérivé N° 2 0,250 g

Excipient : erythrosine, gomme adragante, amidon de blé, talc, lactose, sucre glace.

Le médicament de l'invention peut être présenté pour l'administration orale, sous forme de comprimés, comprimés dragéifiés, capsules, gouttes, granulés ou sirop. Il peut aussi être présenté pour l'administration rectale, sous forme de suppositoires et pour l'administration parentérale sous forme de soluté injectable.

(Voir Tableaux pages 9-14)

Dérivé N° 7

| DOSES EN M/L | TENSION DEVELOPPEE | FREQUENCE DES CONTRACTIONS | CONTRACTION d TENSION / d TEMPS | RELAXATION d TENSION / d TEMPS |
|---|---|---|---|---|
| $10^{-7}$ | 0 % | 0 % | 0 % | 0 % |
| $10^{-6}$ | 5 % | 0 % | 4 % | 16 % |
| $4 \times 10^{-6}$ | 7 % | 0 % | 3 % | 11 % |
| $4 \times 10^{-5}$ | 25 % | 1 % | 31 % | 24 % |
| $4 \times 10^{-4}$ | 76 % | 5 % | 80 % | 69 % |
| $2 \times 10^{-3}$ | 197 % | 3 % | 220 % | 154 % |

Dérivé N° 9

| DOSES EN M/L | TENSION DEVELOPPEE | FREQUENCE DES CONTRACTIONS | CONTRACTION $\frac{d\ TENSION}{d\ TEMPS}$ | RELAXATION $\frac{d\ TENSION}{d\ TEMPS}$ |
|---|---|---|---|---|
| $10^{-6}$ | 9 % | 0 % | 13 % | 10 % |
| $10^{-5}$ | 24 % | 1 % | 20 % | 28 % |
| $5 \times 10^{-5}$ | 26 % | 3 % | 29 % | 36 % |
| $10^{-4}$ | 59 % | 4 % | 51 % | 64 % |
| $5 \times 10^{-4}$ | 86 % | 4 % | 67 % | 53 % |
| $10^{-3}$ | 129 % | 4 % | 107 % | 128 % |

Dérivé N° 12

| DOSES EN M/L | TENSION DEVELOPPEE | FREQUENCE DES CONTRACTIONS | CONTRACTION $\frac{d\ TENSION}{d\ TEMPS}$ | RELAXATION $\frac{d\ TENSION}{d\ TEMPS}$ |
|---|---|---|---|---|
| $10^{-6}$ | 4 % | 0 % | 2 % | 4 % |
| $10^{-5}$ | 27 % | 19 % | 33 % | 34 % |
| $10^{-4}$ | 48 % | 6 % | 55 % | 43 % |
| $5X10^{-4}$ | 73 % | 17 % | 70 % | 48 % |
| $10^{-3}$ | 145 % | 19 % | 153 % | 140 % |

Dérivé N° 16

| DOSES EN M/L | TENSION DEVELOPPEE | FREQUENCE DES CONTRACTIONS | CONTRACTION d TENSION / d TEMPS | RELAXATION d TENSION / d TEMPS |
|---|---|---|---|---|
| $10^{-6}$ | - 2 % | 0 % | - 10 % | 0 % |
| $10^{-5}$ | 0 % | 0 % | 0 % | 7 % |
| $10^{-4}$ | 39 % | 5 % | 28 % | 31 % |
| $5X10^{-4}$ | 43 % | 13 % | 43 % | 52 % |
| $10^{-3}$ | 130 % | 4 % | 136 % | 119 % |

Dérivé N° 17

| DOSES EN M/L | TENSION DEVELOPPEE | FREQUENCE DES CONTRACTIONS | CONTRACTION $\frac{d\ TENSION}{d\ TEMPS}$ | RELAXATION $\frac{d\ TENSION}{d\ TEMPS}$ |
|---|---|---|---|---|
| $10^{-6}$ | 9 % | 2 % | 16 % | 13 % |
| $10^{-5}$ | 27 % | 6 % | 26 % | 27 % |
| $10^{-4}$ | 45 % | 10 % | 35 % | 46 % |
| $5 \times 10^{-4}$ | 67 % | 14 % | 63 % | 45 % |
| $10^{-3}$ | 110 % | 6 % | 108 % | 114 % |

Dérivé N° 27

| DOSES EN M/L | TENSION DEVELOPPEE | FREQUENCE DES CONTRACTIONS | CONTRACTION d TENSION / d TEMPS | RELAXATION d TENSION / d TEMPS |
|---|---|---|---|---|
| $10^{-6}$ | - 3 % | 4 % | 0 % | 0 % |
| $10^{-5}$ | 10 % | 8 % | 18 % | 10 % |
| $10^{-4}$ | 32 % | 17 % | 31 % | 48 % |
| $10^{-3}$ | 114 % | 27 % | 103 % | 104 % |

3) Gélules

Dérivé N° 11 0,500 g

Excipient : Talc, acide stéarique, stéarate de magnésium.

4) Suppositoires

Dérivé N° 12 0,200 g

Triglycérides semi-synthétiques q. s. p. 1 suppositoire.

5) Soluté injectable

Dérivé N° 6 0,250 g

Solvant isotonique q. s. p. 2 ml.

Doué d'une action inotrope positive, le médicament de l'invention possède une activité toni-cardiaque importante.

Il est utilisé avec profit chez l'homme et chez l'enfant dans l'insuffisance cardiaque aiguë et les accidents paroxystiques de l'insuffisance cardiaque, dans certains troubles du rythme avec risque de décompensation imminente, dans les défaillances cardiaques, et dans les insuffisances cardiaques congestives.

**Revendications** (pour les Etats contractants : BE, CH, DE, FR, GB, IT, LI, LU, NL, SE)

1. Dérivés de triazolo pyrimidine répondant à la formule suivante :

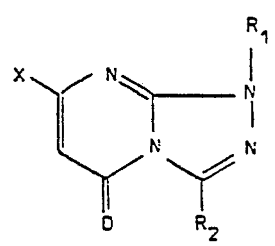

(I)

dans laquelle X représente un noyau pyridyle éventuellement substitué par au moins un groupe alcoyle ou alcoxy en $C_1$ à $C_4$ à chaîne droite ou ramifiée, hydroxy ou cyano, $R_1$ représente l'hydrogène ou un groupe alcoyle en $C_1$ à $C_5$ à chaîne droite ou ramifiée, allyle ou propargyle, et $R_2$ représente un groupe alcoyle en $C_1$ à $C_4$ à chaîne droite ou ramifiée, ainsi que les sels d'addition avec les acides minéraux ou organiques pharmaceutiquement acceptables.

2. Dérivés selon la revendication 1, caractérisés en ce que le noyau pyridyle est un noyau pyridyle-2, pyridyle-3 ou pyridyle-4.

3. Ethyl-1 méthyl-3 oxo-5 (pyridyl-3)-7 triazolo [4,3-a] pyrimidine.

4. Ethyl-1 méthyl-3 oxo-5 (pyridyl-4)-7 triazolo [4,3-a] pyrimidine.

5. Procédé de préparation des dérivés de formule I, caractérisé en ce qu'on condense, dans un solvant organique, un dérivé de formule II :

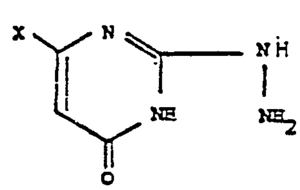

(II)

dans lequel X est tel que défini à la revendication 1 avec des ortho esters de formule III

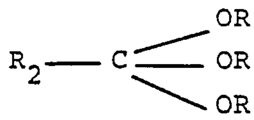

(III)

dans lesquels $R_2$ est tel que défini à la revendication 1 et R représente le groupe méthyle ou éthyle et on procède ensuite éventuellement à une alcoylation, en présence d'une base dans un solvant organique, pour obtenir les composés dans lesquels $R_1$ n'est pas l'hydrogène, les sels d'acides minéraux ou organiques pharmaceutiquement acceptables étant ensuite préparés par les méthodes classiques.

6. Procédé selon la revendication 5 caractérisé en ce que le solvant organique de la réaction de condensation est l'ortho ester lui-même, ou le xylène, le butanol ou le toluène.

7. Procédé selon la revendication 5 caractérisé en ce que d'une base, dans le solvant organique de la réaction d'alcoylation est le diméthyl formamide, le diméthyl sulfoxyde, l'hexaméthylphosphorotriamide, l'acétonitrile ou le toluène.

8. Médicament présentant notamment une activité tonicardiaque caractérisé en ce qu'il contient, à titre de principe actif un composé selon les revendications 1 à 4.

9. Médicament selon la revendication 8 caractérisé en ce qu'il est présenté sous une forme appropriée à l'administration orale, parentérale ou rectale, le principe actif étant associé à un véhicule thérapeutiquement acceptable.

10. Médicament selon la revendication 8 caractérisé en ce qu'il est présenté sous forme de doses unitaires contenant chacune de 0,050 g à 1,00 g.

**Revendications** (pour l'Etat contractant AT)

1. Procédé de préparation de dérivés de triazolo pyrimidine répondant à la formule suivante :

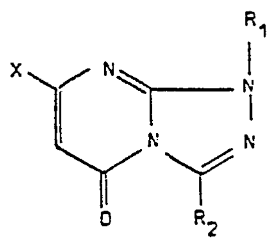

(I)

dans laquelle X représente un noyau pyridyle éventuellement substitué par au moins un groupe alcoyle ou alcoxy en $C_1$ à $C_4$ à chaîne droite ou ramifiée, hydroxy ou cyano, $R_1$ représente l'hydrogène ou un groupe alcoyle en $C_1$ à $C_5$ à chaîne droite ou ramifiée, allyle ou propargyle, et $R_2$ représente un groupe alcoyle en $C_1$ à $C_4$ à chaîne droite ou ramifiée, ainsi que les sels d'addition avec les acides minéraux ou organiques pharmaceutiquement acceptables, procédé caractérisé en ce qu'on condense, dans un solvant organique, un dérivé de formule II :

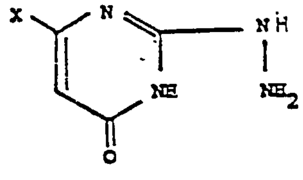

(II)

dans lequel X est tel que défini pour la formule I avec des ortho esters de formule III

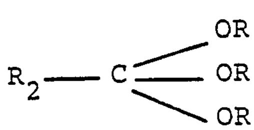

(III)

dans lesquels $R_2$ est tel que défini pour la formule I et R représente le groupe méthyle ou éthyle et on procède ensuite éventuellement à une alcoylation, en présence d'une base dans un solvant organique,

pour obtenir les composés dans lesquels $R_1$ n'est pas l'hydrogène, les sels d'acides minéraux ou organiques pharmaceutiquement acceptables étant ensuite préparés par les méthodes classiques.

2. Procédé selon la revendication 1, caractérisé en ce que le noyau pyridyle est un noyau pyridyle-2, pyridyle-3 ou pyridyle-4.

3. Procédé selon la revendication 2, caractérisé en ce qu'on prépare l'éthyl-1 méthyl-3 oxo-5 (pyridyl-3)-7 triazolo [4,3-a] pyrimidine.

4. Procédé selon la revendication 1, caractérisé en ce que l'on prépare l'éthyl-1 méthyl-3 oxo-5 (pyridyl-4)-7 triazolo [4,3-a] pyrimidine.

5. Procédé selon la revendication 1, caractérisé en ce que le solvant organique de la réaction de condensation est l'ortho ester lui-même, ou le xylène, le butanol ou le toluène.

6. Procédé selon la revendication 1, caractérisé en ce que le solvant organique de la réaction d'alcoylation est le diméthyl formamide, le diméthyl sulfoxyde, l'hexaméthylphosphorotriamide, l'acétonitrile ou le toluène.

7. Procédé de préparation d'un médicament présentant notamment une activité tonicardiaque, caractérisé en ce qu'on utilise, à titre de principe actif un composé obtenu selon les revendications 1 à 4.

**Claims** (for the Contracting States : BE, CH, DE, FR, GB, IT, LI, LU, NL, SE)

1. Triazolo pyrimidine derivatives corresponding to the following formula :

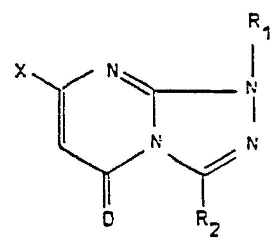

(I)

wherein X represents a pyridyl nucleus optionally substituted by at least one straight-chained or branched $C_{1-4}$ alkoxy or alkyl group or a hydroxy or cyano group, $R_1$ represents hydrogen or a straight-chained or branched $C_{1-5}$ alkyl, an allyl or a propargyl group and $R_2$ represents a straight-chained or banched $C_{1-4}$ alkyl group, and the pharmaceutically acceptable acid addition salts thereof with inorganic or organic acids.

2. Derivatives as claimed in claim 1, characterised in that the pyridyl nucleus is a 2-pyridyl, 3-pyridyl or 4-pyridyl nucleus.

3. 1-Ethyl-3-methyl-5-oxo-7-(3-pyridyl)-triazolo [4,3-a] pyrimidine.

4. 1-Ethyl-3-methyl-5-oxo-7-(4-pyridyl)-triazolo-[4,3-a] pyrimidine.

5. Process for preparing derivatives of formula I, characterised in that a derivative of formula II :

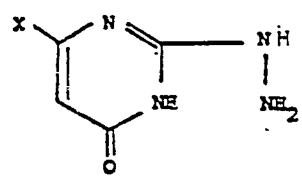

(II)

wherein X is as defined in claim 1 is condensed in an organic solvent with orthoesters of formula III

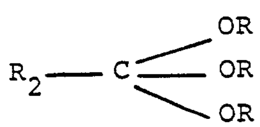

(III)

wherein $R_2$ is as defined in claim 1 and R represents a methyl or ethyl group and subsequently, if desired, alkylation is carried out in the presence of a base in an organic solvent in order to obtain compounds wherein $R_1$ is not hydrogen, then the pharmaceutically acceptable addition salts with inorganic or organic acids are prepared by conventional methods.

6. Process as claimed in claim 5, characterised in that the organic solvent of the reaction of condensation is the orthoester itself or xylene, butanol or toluene.
7. Process as claimed in claim 5, characterised in that the organic solvent of the reaction of alkylation is dimethylformamide, dimethylsulphoxide, hexamethylphosphorotriamide, acetonitrile or toluene.
8. Medicament having, in particular, a cardiotonic activity, characterised in that it contains as active ingredient a compound as claimed in claims 1 and 2.
9. Medicament as claimed in claim 8, characterised in that it is presented in a form suitable for oral, parenteral or rectal administration, the active ingredient being combined with a therapeutically acceptable carrier.
10. Medicament as claimed in claim 8, characterised in that it is presented in the form of dosage units each containing from 0.050 g to 1.00 g.

**Claims** (for the Contracting State AT)

1. Process for preparing triazolo pyrimidine derivatives corresponding to the following formula :

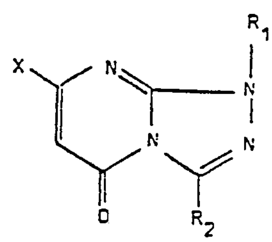

(I)

wherein X represents a pyridyl nucleus optionally substituted by at least one straight-chained or branched $C_{1-4}$ alkoxy or alkyl group or a hydroxy or cyano group, $R_1$ represents hydrogen or a straight-chained or branched $C_{1-5}$ alkyl, an allyl or a propargyl group and $R_2$ represents a straight-chained or branched $C_{1-4}$ alkyl group, and the pharmaceutically acceptable acid addition salts thereof with inorganic or organic acids, characterised in that a derivative of formula II

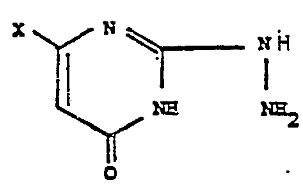

(II)

wherein X is as defined in claim 1 is condensed in an organic solvent with orthoesters of formula III

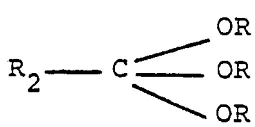

(III)

wherein $R_2$ is as defined in claim 1 and R represents a methyl or ethyl group and subsequently, if desired, alkylation is carried out in the presence of a base in an organic solvent in order to obtain compounds wherein $R_1$ is not hydrogen, then the pharmaceutically acceptable addition salts with inorganic or organic acids are prepared by conventional methods.
2. Process as claimed in claim 1, characterised in that the pyridyl nucleus is a 2-pyridyl, 3-pyridyl or 4-pyridyl nucleus.
3. Process as claimed in claim 2, characterised in that 1-ethyl-3-methyl-5-oxo-7-(3-pyridyl)-triazolo [4,3-a] pyrimidine is prepared.
4. Process as claimed in claim 1, characterised in that 1-ethyl-3-methyl-5-oxo-7-(4-pyridyl)-triazolo-[4,3-a] pyrimidine is prepared.
5. Process as claimed in claim 1, characterised in that the organic solvent of the reaction of condensation is the orthoester itself or xylene, butanol or toluene.
6. Process as claimed in claim 1, characterised in that the organic solvent of the reaction of

alkylation is dimethylformamide, dimethylsulphoxide, hexamethylphosphorotriamide, acetonitrile or toluene.

7. Process for preparing a medicament having, in particular, a cardiotonic activity, characterised in that a compound obtained according to claims 1 to 4 is rised as active ingredient.

**Patentansprüche** (für die Vertragsstaaten : BE, CH, DE, FR, GB, IT, LI, LU, NL, SE)

1. Triazolopyrimidinderivate entsprechend der folgenden Formel :

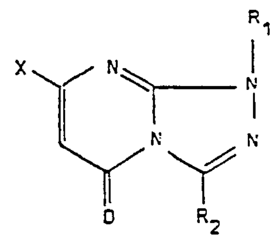

(I)

worin X ein Pyridylkern ist, der gegebenenfalls durch wenigstens eine geradkettige oder verzweigte $C_1$-$C_4$-Alkyl- oder Alkoxygruppe, Hydroxy oder Cyano substituiert ist, $R_1$ Wasserstoff oder eine geradkettige oder verzweigte $C_1$-$C_5$-Alkylgruppe, eine Allyl- oder Propargylgruppe darstellt und $R_2$ eine geradkettige oder verzweigte $C_1$-$C_4$-Alkylgruppe darstellt, sowie die Additionssalze mit pharmazeutisch unbedenklichen anorganischen oder organischen Säuren.

2. Derivate nach Anspruch 1, dadurch gekennzeichnet, daß der Pyridylkern ein 2-Pyridyl-, 3-Pyridyl- oder 4-Pyridylkern ist.

3. 1-Ethyl-3-methyl-5-oxo-7-(3-pyridyl)-triazolo [4,3-a] pyrimidin.

4. 1-Ethyl-3-methyl-5-oxo-7-(4-pyridyl)-triazolo [4,3-a] pyrimidin.

5. Verfahren zur Herstellung der Derivate der Formel I, dadurch gekennzeichnet, daß man in einem organischen Lösungsmittel ein Derivat der Formel II :

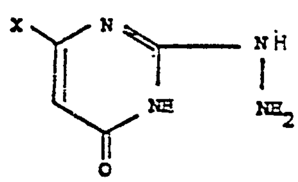

(II)

in der X die in Anspruch 1 definierte Bedeutung hat, mit Orthoestern der Formel III

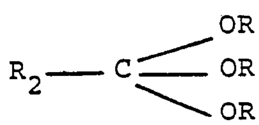

(III)

worin $R_2$ die in Anspruch 1 definierte Bedeutung hat und R die Methyl- oder Ethylgruppe ist, kondensiert und anschließend gegebenenfalls eine Alkylierung in Gegenwart einer Base in einem organischen Lösungsmittel durchführt, um die Verbindungen zu erhalten, in denen $R_1$ kein Wasserstoff ist, wobei die Salze von pharmazeutisch unbedenklichen anorganischen oder organischen Säuren anschließend nach den klassischen Methoden hergestellt werden.

6. Verfahren nach Anspruch 5, dadurch gekennzeichnet, daß das organische Lösungsmittel der Kondensationsreaktion der Orthoester selbst oder Xylol, Butanol oder Toluol ist.

7. Verfahren nach Anspruch 5, dadurch gekennzeichnet, daß das organische Lösungsmittel der Alkylierungsreaktion Dimethylformamid, Dimethylsulfoxid, Hexamethylphosphorotriamid, Acetonitril oder Toluol ist.

8. Arzneimittel mit insbesondere kardiotonischer Wirkung, dadurch gekennzeichnet, daß es als Wirkstoff eine Verbindung nach den Ansprüchen 1 bis 4 enthält.

9. Arzneimittel nach Anspruch 8, dadurch gekennzeichnet, daß es in einer für die orale, parenterale oder rektale Verabreichung geeigneten Form vorliegt, wobei der Wirkstoff mit einem therapeutisch unbedenklichen Träger assoziiert ist.

10. Arzneimittel nach Anspruch 8, dadurch gekennzeichnet, daß es in Einheitsdosen vorliegt, die jeweils 0,050 bis 1,00 g enthalten.

**Patentansprüche** (für den Vertragsstaat AT)

1. Verfahren zur Herstellung von Triazolopyrimidinderivaten der folgenden Formel :

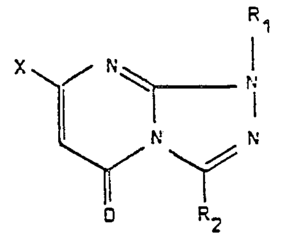

(I)

worin X ein Pyridylkern ist, der gegebenenfalls durch wenigstens eine geradkettige oder verzweigte $C_1$-$C_4$-Alkyl- oder Alkoxygruppe, Hydroxy oder Cyano substituiert ist, $R_1$ Wasserstoff oder eine geradkettige oder verzweigte $C_1$-$C_5$-Alkylgruppe, eine Allyl- oder Propargylgruppe darstellt und $R_2$ eine geradkettige oder verzweigte $C_1$-$C_4$-Alkylgruppe darstellt, sowie der Additionssalze mit pharmazeutisch unbedenklichen anorganischen oder organischen Säuren, dadurch gekennzeichnet, daß man in einem organischen Lösungsmittel ein Derivat der Formel II :

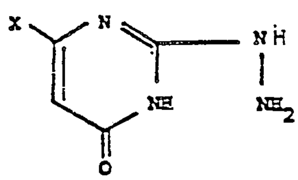

(II)

in der X die für die Formel I definierte Bedeutung hat, mit Orthoestern der Formel III

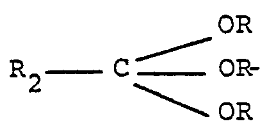

(III)

worin $R_2$ die für die Formel I definierte Bedeutung hat und R die Methyl- oder Ethylgruppe darstellt, kondensiert und anschließend gegebenenfalls eine Alkylierung in Gegenwart einer Base in einem organischen Lösungsmittel vornimmt, um die Verbindungen zu erhalten, in denen $R_1$ kein Wasserstoff ist, und die Salze von pharmazeutisch unbedenklichen anorganischen oder organischen Säuren anschließend nach klassischen Methoden herstellt.

2. Verfahren nach Anspruch 1, dadurch gekennzeichnet, daß der Pyridylkern ein 2-Pyridyl-, 3-Pyridyl- oder 4-Pyridylkern ist.

3. Verfahren nach Anspruch 2, dadurch gekennzeichnet, daß man 1-Ethyl-3-methyl-5-oxo-7-(3-pyridyl)-triazolo [4,3-a] pyrimidin herstellt.

4. Verfahren nach Anspruch 1, dadurch gekennzeichnet, daß man 1-Ethyl-3-methyl-5-oxo-7-(4-pyridyl)-triazolo [4,3-a] pyrimidin herstellt.

5. Verfahren nach Anspruch 1, dadurch gekennzeichnet, daß das organische Lösungsmittel der Kondensationsreaktion der Orthoester selbst oder Xylol, Butanol oder Toluol ist.

6. Verfahren nach Anspruch 1, dadurch gekennzeichnet, daß das organische Lösungsmittel der Alkylierungsreaktion Dimethylformamid, Dimethylsulfoxid, Hexamethylphosphorotriamid, Acetonitril oder Toluol ist.

7. Verfahren zur Herstellung eines Arzneimittels, das insbesondere eine kardiotonische Wirkung aufweist, dadurch gekennzeichnet, daß man als Wirkstoff eine gemäß den Ansprüchen 1 bis 4 erhaltene Verbindung verwendet.